# FASCICULE DE BREVET EUROPEEN

(11) **EP 2 677 976 B1**
(45) Date de publication et mention de la délivrance du brevet: **12.11.2014**
(21) Numéro de dépôt: 12707858.2
(22) Date de dépôt: 03.02.2012
(51) Int. Cl.: A61F 5/01

(54) **Orthèse pour le traitement des troubles musculaires du coude**
Orthese zur Behandlung von Muskelstörungen im Ellenbogen
Orthosis for treating muscle disorders in the elbow

(30) Priorité: 21.02.2011 FR 1151405
(43) Date de publication de la demande: 01.01.2014
(73) Titulaire: Gibaud, 42000 Saint Etienne (FR)
(72) Inventeur: ANGLADA, Gérard, F-42000 Saint Etienne (FR); GIRARD, Frédéric, F-75009 Paris (FR)
(74) Mandataire: Delorme, Nicolas
(86) Numéro de dépôt international: PCT/FR2012/050240
(87) Numéro de publication internationale: WO 2012/114012

(56) Documents cités:
- US-A- 5 836 902
- US-A1- 2004 024 339
- US-A1- 2006 089 583
- US-A1- 2008 034 459

## Description

La présente invention concerne une orthèse pour le traitement des troubles musculaires du coude, qui est apte à fournir une assistance aux muscles extenseurs du poignet.

L'un des troubles musculaires plus spécifiquement visés par la présente invention est l'épicondylite. Il s'agit d'une tendinite qui se manifeste par des douleurs au coude et qui est souvent due à une trop grande sollicitation des muscles de l'avant-bras. Ce trouble peut notamment apparaître suite à des mouvements répétitifs, mal exécutés ou particulièrement durs de la main et/ou du poignet, que ce soit dans le travail (menuisiers, maçons, opérateurs de marteaux-piqueurs, etc.) ou lors de la pratique d'un sport (tennis, golf, etc.) ou d'un loisir (par exemple le jardinage). Ainsi, l'épicondylite apparaît lorsque le tendon des muscles extenseurs est trop sollicité ; la douleur se situe alors surtout dans la partie externe de l'avant-bras, dans la région de l'épicondyle, qui est une petite saillie osseuse de la face externe de l'humérus.

Ce trouble provoque des douleurs pouvant durer quelques semaines, voire se prolonger davantage, et, s'ils sont mal soignés, peuvent dégénérer en douleurs chroniques et causer des lésions irréversibles.

Au début de la crise, il est préférable de mettre le coude au repos au moins partiel, en évitant ou au moins en maîtrisant les gestes qui ont causé la lésion. Ultérieurement, en phase de réadaptation, il pourra être possible de reprendre progressivement les mouvements, de préférence en portant un dispositif de serrage de l'avant-bras, qui permet de limiter les sollicitations subies par le tendon en comprimant localement le muscle.

Les documents US 2008/0034459, US 5 836 902 et US 2006/0089583 décrivent des orthèses de poignet. Plus spécifiquement, le document US 2004/0024339 décrit une orthèse selon le préambule de la revendication 1.

La présente invention vise à fournir une orthèse utilisable en phase aigue d'une épicondylite, notamment, qui permette de soulager et de traiter l'utilisateur, tout en lui permettant, le cas échéant, d'effectuer un certain nombre de mouvements non néfastes dans le cadre des troubles musculaires concernés.

L'invention concerne donc une orthèse pour le traitement des troubles musculaires du coude, tels que l'épicondylite, apte à fournir une assistance aux muscles extenseurs du poignet, selon la revendication 1.

En pratique, tout d'abord, le mouvement de pronosupination, qui est générateur de douleurs dans le cas des troubles visés par l'invention, est empêché, ou du moins restreint, grâce à la présence sur le corps de l'orthèse des ailes et branches latérales.

Par ailleurs, en position de repos - c'est-à-dire à l'état non contraint du corps de l'orthèse - le poignet est maintenu en extension, ce qui permet de soulager les muscles extenseurs. Par exemple, le poignet est maintenu selon une extension de l'ordre de 20 à 40°, typiquement de l'ordre de 30°.

A partir de la position de repos, l'utilisateur peut fléchir le poignet, à l'encontre de la résistance à la déformation du corps de l'orthèse. Cette flexion implique donc un certain effort, mais celui-ci est limité du fait du caractère élastiquement déformable de la bande de jonction. De plus, dans le cas des troubles visés par l'invention, le mouvement de flexion du poignet n'est en général ni néfaste ni douloureux.

Une fois le poignet fléchi, en particulier pour le retour à la position de repos, l'extension du poignet est accompagnée par l'effet de rappel élastique du corps de l'orthèse. En conséquence, les muscles extenseurs ne sont pas sollicités, ou le sont peu.

Il est à noter que, avec l'orthèse selon l'invention, c'est pour l'essentiel, voire uniquement, la bande de jonction qui se déforme, et non les branches ou ailes latérales.

Un autre avantage de l'invention réside dans la géométrie « dorso-palmaire » de l'orthèse : le fait que les parties semi rigides de l'orthèse sont placées respectivement sur l'avant-bras et sous la main permet d'obtenir un effet bras de levier qui garantit un excellent maintien de l'orthèse sans compression excessive. Cette disposition permet de répartir la contrainte sur une superficie plus importante de la main et de l'avant-bras de l'utilisateur sans qu'il soit nécessaire de beaucoup serrer l'orthèse. En complément, avantageusement, on peut prévoir que la sangle passe uniquement sous la face antérieure de l'avant-bras, c'est-à-dire pas sur la face postérieure de l'avant-bras.

Par « semi rigide », on entend que le corps de l'orthèse possède à la fois une rigidité suffisante pour adopter une géométrie donnée à l'état non contraint, et procurer le maintien souhaité en extension, et une certaine capacité à être déformée élastiquement pour autoriser la flexion.

Ainsi, l'invention fournit une orthèse dynamique qui permet, de façon quasiment libre, l'accomplissement de certains mouvements non délétères. Un premier avantage correspondant est que le port de l'orthèse est moins contraignant au quotidien, ce qui fait qu'un utilisateur est plus enclin à porter cette orthèse. Ceci permet d'éviter la chronicité de l'inflammation en limitant la sollicitation des muscles épicondyliens. Un autre avantage est d'éviter une immobilisation prolongée qui, dans certains cas, pourrait provoquer une raideur grave de l'articulation, parfois irréversible. De plus, l'orthèse permet également l'accomplissement des mouvements néfastes ou douloureux, mais de façon accompagnée, ce qui leur ôte leur caractère délétère et offre à l'utilisateur une relative liberté au quotidien.

Selon une réalisation possible, l'orthèse est dépourvue d'organe venant en appui contre la face dorsale de la main, tel qu'une sangle ou équivalent. En effet, la géométrie « dorso-palmaire » de l'orthèse suffit à assurer un très bon maintien de cette orthèse sans nécessiter un appui supplémentaire sur la face dorsale de la main, qui pourrait engendrer des problèmes de serrage excessif, nuire au confort de port et/ou entraver les mouvements du poignet.

L'orthèse peut en outre comporter des moyens de rigidification conçus pour pouvoir empêcher la déformation du corps de l'orthèse lorsqu'une pression est exercée sur l'appui palmaire à l'opposé de la bande de jonction. L'orthèse permet ainsi une immobilisation du poignet en extension et empêche la flexion du poignet. Ceci peut être utilisé notamment en phase grave des troubles visés par l'invention.

Selon une réalisation possible, les moyens de rigidification comprennent une pièce rigide pouvant être fixée de façon amovible sur la bande de jonction de façon à empêcher la déformation élastique de cette bande de jonction.

En variante, ou en complément, les moyens de rigidification peuvent comprendre au moins un organe rigide monté sur la partie proximale, respectivement sur la partie distale, du corps de l'orthèse de façon pivotante autour d'un axe sensiblement transversal, entre une position active dans laquelle l'organe est accroché à la partie distale, respectivement à la partie proximale, par des moyens de retenue, pour empêcher la déformation du corps de l'orthèse, et une position inactive, dans laquelle l'organe autorise la déformation du corps de l'orthèse. Ces moyens de rigidification sont donc imperdables.

Un tel organe peut comprendre une patte latérale possédant une première extrémité qui est montée sur une aile latérale de la partie proximale, respectivement une branche latérale de la partie distale, du corps de l'orthèse, et une deuxième extrémité qui est pourvue de moyens de retenue amovible sur ladite branche latérale et sur ladite aile latérale, en position active ou inactive. De façon concrète, si la première extrémité de la patte est montée pivotante sur la partie proximale, la deuxième extrémité de la patte peut être maintenue sur la branche latérale en position active, et sur l'aile latérale en position inactive.

On décrit à présent, à titre d'exemples non limitatifs, plusieurs modes de réalisation possibles de l'invention, en référence aux figures annexées :
La figure 1 est une vue en perspective d'une orthèse selon un mode de réalisation de l'invention, avec des moyens de rigidification en position active ;
La figure 2 est une vue similaire à la figure 1, les moyens de rigidification étant en position inactive ;
La figure 3 est une vue de dessous de l'orthèse de la figure 1 ;
Les figures 4 à 6 montrent une orthèse portée par un utilisateur, respectivement en vue latérale, en vue postérieure et en vue antérieure ;
La figure 7 illustre le pivotement des moyens de rigidification de l'orthèse de la figure 1, de la position active à la position inactive ;
La figure 8 illustre le mouvement de flexion du poignet autorisé par l'orthèse ;
La figure 9 illustre le mouvement d'extension du poignet assisté par l'orthèse ;
Les figures 10 à 12 sont des vues en perspective d'une l'orthèse selon l'invention, équipée de moyens de rigidification selon trois modes de réalisation différents ;
Les figures 13 et 14 sont des vues partielles de l'orthèse de la figure 12 en coupe longitudinale, montrant les moyens de rigidification en position montée et en cours de désolidarisation du corps de l'orthèse.

Comme illustré sur les figures, une orthèse 1 selon l'invention comprend un corps 2 qui est réalisé d'une seule pièce en un matériau semi rigide, par exemple en polyamide. Avantageusement, on peut prévoir que le corps 2 de l'orthèse 1 soit transparent ou translucide, de façon à obtenir un produit discret à porter. Ceci s'ajoute à la légèreté d'aspect et au faible poids de l'orthèse 1, qui peut être portée sous un vêtement.

Le corps 2 comporte une partie distale 3 destinée à être placée au niveau de la main 4 d'un utilisateur et une partie proximale 5 destinée à être placée au niveau de l'avant-bras 6.

On définit la direction longitudinale D1 comme la direction générale dans laquelle s'étend l'avant-bras (lorsque l'orthèse 1 est portée), la direction transversale D2 comme la direction de l'articulation du poignet en flexion / extension et la direction verticale D3 comme la direction de l'articulation du poignet en adduction / abduction. Les termes « proximal » et « distal » sont employés en référence à la direction D1 et le terme « latéral » en référence à la direction D2.

La partie distale 3 comporte un appui palmaire 7 sensiblement transversal pour la paume 8 de la main 4, prolongé dans la direction proximale par deux branches latérales 9 sensiblement longitudinales qui sont jointes à leur extrémité proximale par un pont distal 10 sensiblement transversal, qui est destiné à venir coiffer la face postérieure du poignet.

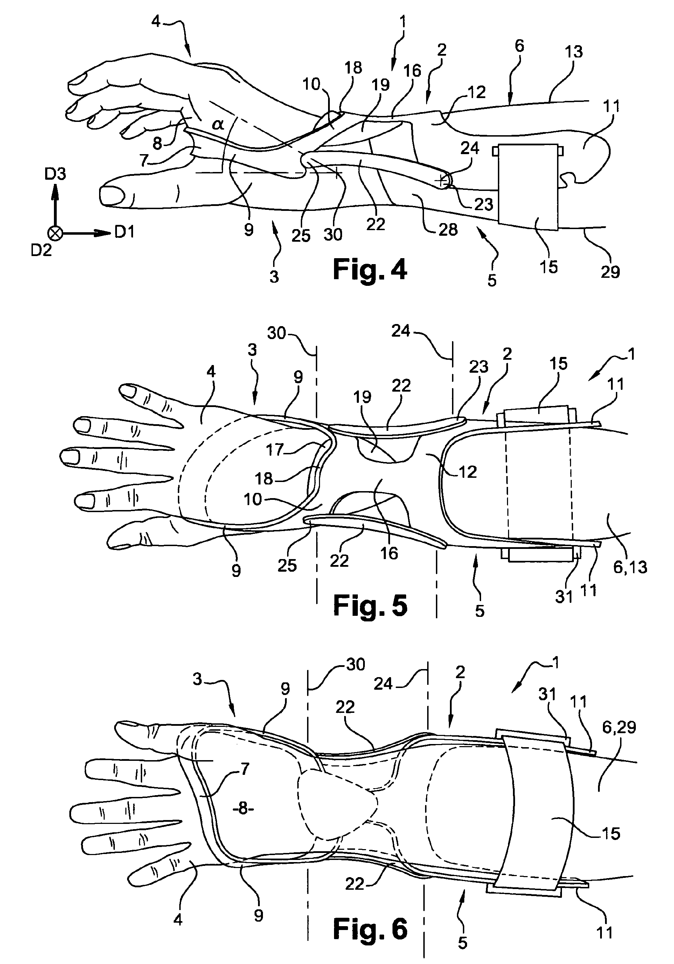

La partie proximale 5 comporte deux ailes latérales 11 sensiblement longitudinales destinées à venir en appui de part et d'autre de l'avant-bras 6 et qui sont jointes à leur extrémité distale par un pont proximal 12 sensiblement transversal destiné à venir coiffer la face postérieure 13 de l'avant-bras 6. Chaque aile 11 peut comporter un orifice 14 de passage d'une sangle 15, les orifices 14 s'étendant de préférence sensiblement longitudinalement. La sangle 15 peut passer dans des passants 31 situés chacun sur une aile latérale 11 (voir notamment la figure 6). La localisation de ces passants 31 permet un coulissement de la sangle 15 sans pincement cutanée.

De plus, le corps 2 de l'orthèse 1 comprend une bande dorsale de jonction 16 s'étendant sensiblement longitudinalement et reliant le pont distal 10 et le pont proximal 12. La partie distale 3, la partie proximale 5 et la bande dorsale de jonction 16 sont réalisées d'une seule pièce.

On peut prévoir que le pont distal 10 comprenne une échancrure 17 ménagée à son bord distal 18, ladite échancrure 17 étant située dans la zone de la styloïde cubitale lorsque l'orthèse 1 est portée. Cette disposition permet d'éviter les douleurs liées à l'appui de l'orthèse 1 sur la styloïde cubitale.

L'orthèse 1 peut en outre comprendre un coussin 19 fixé sous la partie dorsale - ou postérieure - du corps 2 de l'orthèse 1, sous au moins une partie du pont distal 10 et de la bande de jonction 16, de façon que le coussin 19 puisse être interposé entre le poignet de l'utilisateur et le corps 2 de l'orthèse 1. Ainsi, le coussin 19 peut notamment venir en appui sur la styloïde cubitale, ce qui permet de résoudre les éventuels problèmes de positionnement de l'échancrure 17, selon la morphologie de l'utilisateur, et ce bien que l'orthèse 1 puisse exister en plusieurs tailles. Le coussin 19 permet donc d'éviter un éventuel appui douloureux sur la styloïde cubitale. Plus précisément, le coussin 19 peut être fixé sur le corps 2 de l'orthèse 1 de façon à venir en appui dans la zone dorsale de la radio-carpienne. De plus, le coussin 19 permet de reporter l'appui du corps 2 de l'orthèse 1 sur une surface - qui de plus est avantageusement relativement souple - plutôt que sur une arête - à savoir le bord distal 18 du pont distal 10. Il a donc pour effet de répartir la pression.

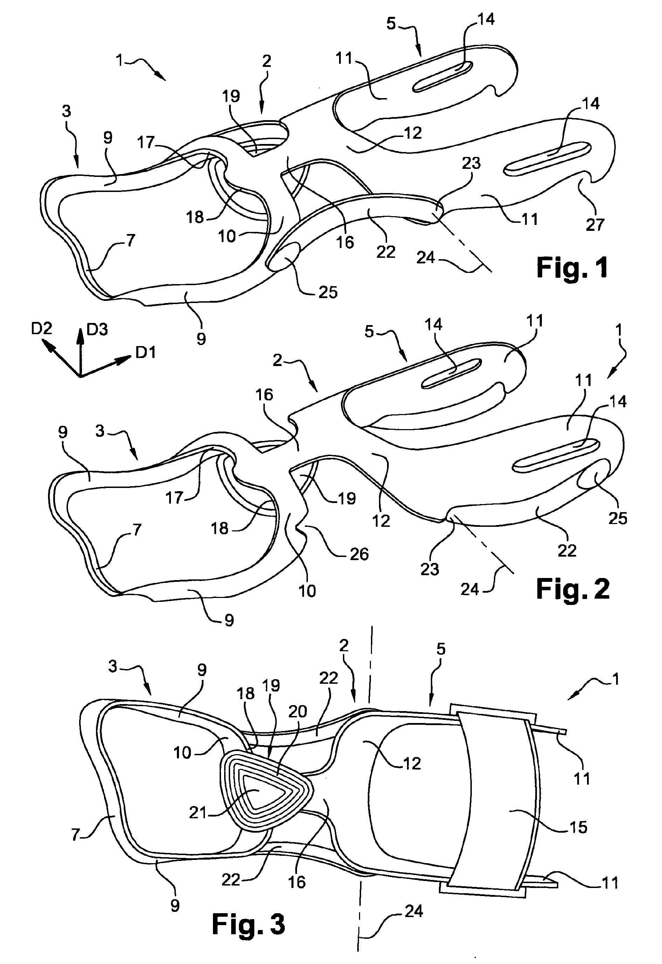

Comme illustré sur la figure 3, le coussin 19 peut comporter au moins une saillie 20 en relief s'étendant sensiblement transversalement, et tournée vers la main lorsque l'orthèse 1 est portée. Ceci permet notamment d'éviter un glissement de l'orthèse 1 vers l'avant. Le coussin 19 présente par exemple une forme globalement ovale ou triangulaire, comportant sur sa face 21 tournée vers la main 4 plusieurs saillies 20 qui suivent le contour du coussin 19 et sont agencées de façon concentrique.

Le coussin 19 est de préférence réalisé en un matériau possédant des propriétés anti glisse, tel qu'un silicone ou du SEBS (styrène éthylbutylène styrène). Ceci permet de renforcer l'adhérence du coussin 19, en variante ou en complément de la ou des saillies 20.

Dans le mode de réalisation représenté sur les figures 1 à 9, l'orthèse 1 comprend de plus des moyens de rigidification qui comportent deux pattes latérales 22 rigides.

Chaque patte latérale 22 possède une première extrémité 23 qui est montée à la partie distale d'une aile latérale 11 de la partie proximale 5, de façon pivotante autour d'un axe 24 sensiblement transversal. De plus, chaque patte latérale 22 possède une deuxième extrémité 25 qui est pourvue de moyens de retenue amovible sur la partie proximale de la branche latérale 9 correspondante.

Une patte 22 peut ainsi pivoter, autour de l'axe 24, entre une position active, dans laquelle la deuxième extrémité 25 de la patte 22 est accrochée à la branche latérale 9 (figure 1) et une position inactive, dans laquelle la deuxième extrémité 25 de la patte 22 est accrochée à l'aile latérale 11 (figure 2).

Les moyens de retenue ménagés sur la patte 22 peuvent comporter une saillie apte à venir s'engager soit dans une encoche 26 (ou un orifice) ménagée sur la branche latérale 9, en position active, soit dans une encoche 27 (ou un orifice) ménagée sur l'aile latérale 11, en position inactive. L'orthèse peut donc être utilisée seule mais son action thérapeutique peut être complétée par l'utilisation conjointe d'un coudière.

Comme illustré sur la figure 4, l'orthèse 1 peut de plus comporter une coudière 28 se présentant sous la forme d'un manchon textile. En pratique, lorsque l'orthèse 1 est portée, le corps 2 de l'orthèse 1 est placé à l'extérieur de la coudière 26. En d'autres termes, tout d'abord, la coudière 28 est enfilée pour envelopper le coude et s'étendre de part et d'autre de celui-ci, ce qui permet de limiter les mouvements du coude. Avantageusement, le bord distal de la coudière 28 est situé au voisinage du poignet. De la sorte, au moins la partie proximale 5 du corps 2 et la sangle 15 viennent se placer sur la coudière 28.

En pratique, la mise en place de l'orthèse 1 s'effectue comme suit.

De façon préliminaire, l'utilisateur peut mettre en place la coudière 28. Ensuite, l'utilisateur place sa main 4 dans la partie distale 3, la paume contre l'appui palmaire 7 et la pouce sous cet appui palmaire 7. Lors de cette étape, la main 4 peut être dans l'alignement de l'avant-bras 6, c'est-à-dire ni en flexion ni en extension.

Puis l'utilisateur serre la sangle 15. Ceci a pour effet d'une part de mettre la partie proximale 5 en position au niveau de l'avant-bras 6, c'est-à-dire de rabattre la partie proximale 5 contre l'avant-bras 6, de plaquer la bande de jonction 16 contre le poignet, et de serrer l'une contre l'autre les ailes latérales 11 de la partie proximale 5, celles-ci pouvant présenter une certaine élasticité. L'avant-bras 6 est ainsi serré par ces deux ailes 11 et par la sangle 15. Il s'agit d'une sangle inférieure, c'est-à-dire qui passe uniquement contre la face antérieure 29 de l'avant-bras 6. Le corps 2 de l'orthèse 1 est ainsi stabilisé sur l'avant-bras 6, quelle que soit sa circonférence, et limite les mouvements de pronosupination générateurs de douleur lors d'une épicondylite.

Le serrage de la sangle 15 a d'autre part pour effet de soulever la paume 8 de la main 4, en faisant pivoter le poignet autour de son axe de flexion-extension 30, afin de maintenir le poignet en extension. Puisque la distance -selon D1 - entre l'axe 30 et l'extrémité distale de la partie distale 3 est inférieure à distance -selon D1 - entre l'axe 30 et la sangle 15, cette opération s'effectue sans excès de tension.

Les pattes 22, si elles n'étaient pas déjà en position active, peuvent être placées dans cette position active afin de rendre le corps 2 de l'orthèse 1 sensiblement indéformable et donc d'interdire la flexion du poignet. Cette immobilisation stricte du poignet peut être imposée dans certains cas, par exemple selon la gravité des troubles, notamment au début.

Les figures 4 à 6 montrent l'orthèse 1 portée par un utilisateur dans la position de repos, c'est-à-dire à l'état non contraint du corps 2. La position relative des parties distale 3 et proximale 5 est alors telle que le poignet est maintenu en extension selon un angle α de l'ordre de 30°. De ce fait, la contraction des muscles extenseurs (épicondyliens) et de leurs tendons enflammés n'est plus nécessaire pour maintenir le poignet.

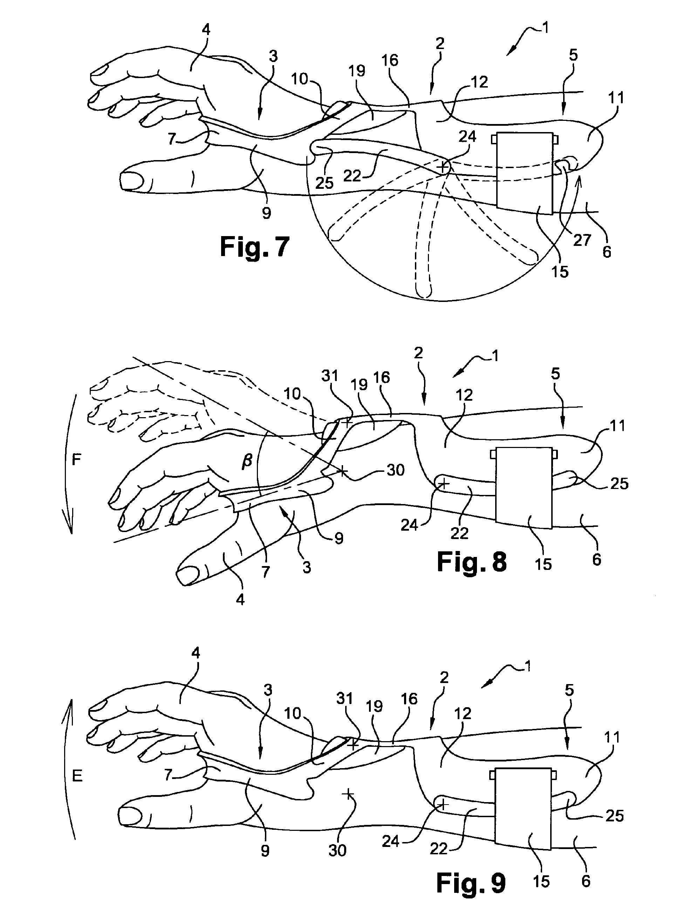

On se reporte à présent aux figures 7 à 9 qui correspondent à une utilisation des propriétés de déformabilité du corps 2.

Comme illustré sur la figure 7, à partir de la position illustrée sur la figure 4, les pattes 22, si elles étaient initialement en position active, sont déplacées par pivotement autour de l'axe 24 pour être rabattues et accrochées chacune sur la branche latérale 11 correspondante, en position inactive. Les pattes 22 sont maintenues entre les parties interne et externe de la sangle 15. La flexion du corps 2 de l'orthèse 1 est alors autorisée.

Malgré le caractère semi-rigide du matériau constitutif du corps 2, du fait de la largeur et de l'épaisseur réduites de la bande de jonction 16, cette dernière peut être déformée élastiquement lorsqu'une pression est exercée sur l'appui palmaire 7 à l'opposé de la bande de jonction 16 - c'est-à-dire vers le bas dans la représentation de la figure 8. Cette déformation de la bande de jonction 16 autorise le pivotement de la partie distale 3 par rapport à la partie proximale 5 du corps autour d'un axe d'articulation 31 sensiblement transversal et parallèle à l'axe 30 de flexion-extension du poignet lorsque l'orthèse 1 est portée. L'axe 31 peut être situé sensiblement à l'intersection de la bande de jonction 16 et du pont distal 10.

Ainsi, l'orthèse 1 permet une certaine flexion du poignet, selon la flèche F de la figure 8, d'un angle β pouvant être de l'ordre de 40 à 60° depuis la position de repose illustrée sur la figure 7.

Comme illustré sur la figure 8, cette flexion du poignet, et déformation du corps 2, se traduit par une courbure de la bande de jonction 16, qui peut localement - typiquement en sa partie centrale - s'éloigner du poignet. Le coussin 19 permet une flexion confortable sans risque de compression du réseau musculo-ligamentaire dorsal du poignet. Cette possibilité de flexion du poignet permet une préhension plus fine (stylo, souris d'ordinateur, conduite avec passage des vitesses, etc.) qui facilite le quotidien de l'utilisateur.

Puis, lorsque les muscles fléchisseurs du poignet se relâchent, le corps 2 revient à l'état non contraint par élasticité, par pivotement de la partie distale 3 par rapport à la partie proximale 5 autour dudit axe d'articulation 31.

La bande de jonction 16 agit ainsi comme une lame ressort qui reprend sa forme et ramène le poignet en extension. Cette extension assistée - selon la flèche E de la figure 9 - évite les tractions répétées des muscles épicondyliens sur leur insertion proximale. Les tendons enflammés ne sont pratiquement plus sollicités, ce qui permet une reprise d'activité sans risque d'épicondylite chronique.

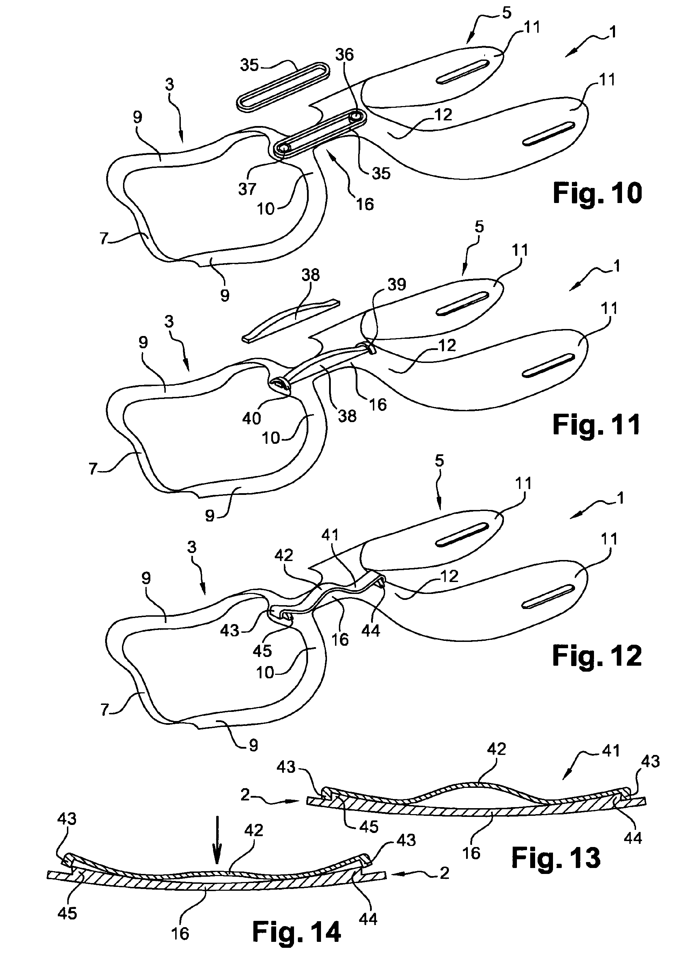

On se réfère maintenant aux figures 10 à 14 qui illustrent d'autres moyens de rigidification conçus pour pouvoir empêcher la déformation du corps 2 de l'orthèse 1 lorsqu'une pression est exercée sur l'appui palmaire 7 à l'opposé de la bande de jonction 16. Ces moyens de rigidification comprennent une pièce rigide pouvant être fixée de façon amovible sur la bande de jonction 16, de façon à empêcher la déformation élastique de cette bande de jonction 16. En retirant la pièce rigide, le corps 2 de l'orthèse 1 peut à nouveau être déformé, lorsque cela est approprié.

Sur la figure 10, la pièce rigide comprend un anneau oblong 35 qui vient se fixer sur un premier téton 36 situé sensiblement à l'intersection entre la bande de jonction 16 et le pont proximal 12 et sur un deuxième téton 37 situé sensiblement à l'intersection entre la bande de jonction 16 et le pont distal 10. L'anneau oblong 35 présente une certaine élasticité pour pouvoir être mis en place sur les tétons 36, 37, tout en empêchant la déformation de la bande de jonction 16.

Sur la figure 11, la pièce rigide comprend une nervure 38 qui vient s'encliqueter sous un premier arc 39 situé sensiblement à l'intersection entre la bande de jonction 16 et le pont proximal 12 et sous un deuxième arc 40 situé sensiblement à l'intersection entre la bande de jonction 16 et le pont distal 10. En position montée, la nervure 38 est située sensiblement dans un plan (D1, D3). Elle présente une certaine élasticité pour pouvoir être mise en place sur les arcs 39, 40, tout en empêchant la déformation de la bande de jonction 16.

Sur la figure 12, la pièce rigide comprend une barrette 41 comportant une partie centrale 42 bombée et un crochet 43 à chacune de ses extrémités, les crochets 43 étant tournées à l'opposé de la partie bombée. Les crochets viennent s'accrocher sur une première saillie 44 située sensiblement à l'intersection entre la bande de jonction 16 et le pont proximal 12 et sur une deuxième saillie 45 située sensiblement à l'intersection entre la bande de jonction 16 et le pont distal 10.

A partir de la position de rigidification (figure 13), où la barrette 41 est accrochée sur les saillies 44, 45 et empêche la déformation de la bande de jonction 16, un utilisateur peut appuyer sur la partie bombée 42 (selon la flèche représentée sur la figure 14). En conséquence, les extrémités de la barrette 41 se soulèvent, libérant ainsi les crochets 43 des saillies 44, 45.

Il va de soi que l'invention n'est pas limitée aux modes de réalisation décrits ci-dessus à titre d'exemples mais qu'elle comprend tous les équivalents techniques et les variantes des moyens décrits ainsi que leurs combinaisons.

## Revendications

1. Orthèse pour le traitement des troubles musculaires du coude, tels que l'épicondylite, apte à fournir une assistance aux muscles extenseurs du poignet, l'orthèse (1) comprenant un corps (2) qui est réalisé d'une seule pièce en un matériau semi rigide et qui comporte :
- une partie distale (3) destinée à être placée au niveau de la main (4), comportant un appui palmaire (7) sensiblement transversal pour la paume (8) de la main, prolongé dans la direction proximale par deux branches latérales (9) sensiblement longitudinales ;
- une partie proximale (5) destinée à être placée au niveau de l'avant-bras (6), comportant deux ailes latérales (11) sensiblement longitudinales destinées à venir en appui de part et d'autre de l'avant-bras ;
une sangle (15) étant associée à chacune des ailes latérales (11) de la partie proximale (5) pour serrer l'une contre l'autre les ailes latérales (11), le corps (2) de l'orthèse (1) présentant une géométrie telle que la position relative des parties distale et proximale (3, 5), à l'état non contraint, correspond à un maintien en extension du poignet lorsque l'orthèse (1) est portée ;
**caractérisée en ce que**
- les deux branches latérales (9) de la partie distale (3) sont jointes à leur extrémité proximale par un pont distal (10) sensiblement transversal destiné à venir coiffer la face postérieure du poignet, et les deux ailes latérales (11) de la partie proximale (5) sont jointes à leur extrémité distale par un pont proximal (12) sensiblement transversal destiné à venir coiffer la face postérieure de l'avant-bras, la sangle (15) passant sous la face antérieure (29) de l'avant-bras ;
- le corps (2) comporte une bande dorsale de jonction (16) qui s'étend sensiblement longitudinalement, qui relie les ponts distal et proximal (10, 12), et qui est élastiquement déformable de sorte que :
- lorsqu'une pression est exercée sur l'appui palmaire (7) à l'opposé de la bande de jonction (16), la bande de jonction (16) se déforme de façon à autoriser le pivotement de la partie distale (3) par rapport à la partie proximale (5) autour d'un axe d'articulation (31) sensiblement transversal et parallèle à l'axe (30) de flexion-extension du poignet lorsque l'orthèse (1) est portée ;
- et lorsque ladite pression est relâchée, le corps (2) revient à l'état non contraint par élasticité, par pivotement de la partie distale (3) par rapport à la partie proximale (5) autour dudit axe d'articulation (31).

2. Orthèse selon la revendication 1, **caractérisée en ce qu'**elle est dépourvue d'organe venant en appui contre la face dorsale de la main (4), tel qu'une sangle ou équivalent.

3. Orthèse selon la revendication 1 ou 2, **caractérisée en ce que** le pont distal (10) comprend une échancrure (17) ménagée à son bord distal (18), ladite échancrure (17) étant située dans la zone de la styloïde cubitale lorsque l'orthèse (1) est portée.

4. Orthèse selon l'une des revendications 1 à 3, **caractérisée en ce qu'**elle comprend en outre un coussin (19) fixé sous la partie dorsale - ou postérieure - du corps (2) de l'orthèse (1), sous au moins une partie du pont distal (10) et de la bande de jonction (16), de façon que le coussin (19) puisse être interposé entre le poignet de l'utilisateur et le corps (2) de l'orthèse (1).

5. Orthèse selon la revendication 4, **caractérisée en ce que** le coussin (19) comporte au moins une saillie (20) en relief s'étendant sensiblement transversalement, et tournée vers la main lorsque l'orthèse (1) est portée.

6. Orthèse selon l'une des revendications 1 à 5, **caractérisée en ce qu'**elle comporte en outre des moyens de rigidification (22, 35, 38, 41) conçus pour pouvoir empêcher la déformation du corps (2) de l'orthèse (1) lorsqu'une pression est exercée sur l'appui palmaire (7) à l'opposé de la bande de jonction (16).

7. Orthèse selon la revendication 6, **caractérisée en ce que** les moyens de rigidification comprennent une pièce rigide (35, 38, 41) pouvant être fixée de façon amovible sur la bande de jonction (16) de façon à empêcher la déformation élastique de cette bande de jonction (16).

8. Orthèse selon la revendication 6 ou 7, **caractérisée en ce que** les moyens de rigidification comprennent au moins un organe rigide (22) monté sur la partie proximale (5), respectivement sur la partie distale (3), du corps (2) de l'orthèse (1) de façon pivotante autour d'un axe (24) sensiblement transversal, entre une position active dans laquelle l'organe (22) est accroché à la partie distale (3), respectivement à la partie proximale (5), par des moyens de retenue, pour empêcher la déformation du corps (2) de l'orthèse (1), et une position inactive, dans laquelle l'organe (22) autorise la déformation du corps (2) de l'orthèse (1).

9. Orthèse selon la revendication 8, **caractérisée en ce que** l'organe comprend une patte latérale (22) possédant une première extrémité (23) qui est montée sur une aile latérale (11) de la partie proximale (5), respectivement une branche latérale (9) de la partie distale (3), du corps (2) de l'orthèse (1), et une deuxième extrémité (25) qui est pourvue de moyens de retenue amovible sur ladite branche latérale (9) et sur ladite aile latérale (11), en position active ou inactive.

10. Orthèse selon l'une des revendications 1 à 9, **caractérisée en ce que** le corps (2) est transparent ou translucide.

11. Orthèse selon l'une des revendications 1 à 10, **caractérisée en ce qu'**elle comprend de plus une coudière (28) se présentant sous la forme d'un manchon textile, le corps (2) de l'orthèse (1) étant placé à l'extérieur de la coudière lorsque l'orthèse (1) est portée.

## Patentansprüche

1. Orthese zur Behandlung von Muskelstörungen im Ellenbogen, wie z.B. Epicondilytis, die dazu in der Lage ist, eine Unterstützung für die Streckermuskel des Handgelenks bereitzustellen, umfassend einen Körper (2), der aus einem einzigen Stück und aus einem halbstarrem Material hergestellt ist, und der Folgendes umfasst:
- einen distalen Teil (3), der ausgelegt ist, um auf der Ebene der Hand (4) positioniert zu werden, umfassend eine palmare Auflage (7), die im Wesentlichen quer liegt, für die Handfläche (8), verlängert in der proximalen Richtung durch zwei seitliche Zweige (9), die im Wesentlichen längs gerichtet sind,
- einen proximalen Teil (5), der dazu ausgelegt ist, um auf der Ebene des Unterarms (6) positioniert zu werden, umfassend zwei seitliche Flügel (11), die im Wesentlichen längs gerichtet sind, die ausgelegt sind, um auf beiden Seiten des Unterarms aufzuliegen,
wobei ein Gurt (15) mit jedem der seitlichen Flügel (11) des proximalen Teils (5) assoziiert ist, um die seitlichen Flügel (11) gegeneinander zu drücken, wobei der Körper (2) der Orthese (1) eine derartige Geometrie aufweist, dass die relative Position des distalen und des proximalen Teils (3, 5) im nicht gespannten Zustand einer Aufrechterhaltung der Streckung des Handgelenks entspricht, wenn die Orthese (1) getragen wird,
**dadurch gekennzeichnet, dass**
- die zwei seitlichen Zweige (9) des distalen Teils (3) an ihrem proximalen Ende durch eine distale Brücke (10) verbunden sind, die im Wesentlichen quer liegt, die ausgelegt ist, um die Rückseite des Handgelenks zu bedecken, und die zwei seitlichen Flügel (11) des proximalen Teils (5) an ihrem distalen Ende durch eine proximale Brücke (12) verbunden sind, die im Wesentlichen quer liegt, die ausgelegt ist, um die Rückseite des Unterarms zu bedecken, wobei der Gurt (15) unter der Vorderseite (29) des Unterarms verläuft;
- der Körper (2) ein Rücken-Verbindungsband (16) umfasst, das sich im Wesentlichen längs erstreckt, das die distale und die proximale Brücke (10, 12) verbindet, und das elastisch verformbar ist, so dass:
- wenn ein Druck auf die palmare Auflage (7) gegenüber dem Verbindungsband (16) ausgeübt wird, sich das Verbindungsband (16) derart verformt, dass es die Drehung des distalen Teils (3) mit Bezug auf den proximalen Teil (5) um eine Gelenkachse (31) ermöglicht, die im Wesentlichen quer und parallel zur Biege-Streck-Achse (30) des Handgelenks ist, wenn die Orthese (1) getragen wird;
- und wenn der Druck gelockert wird, der Körper (2) in den nicht durch die Elastizität gespannten Zustand durch die Drehung des distalen Teils (3) mit Bezug auf den proximalen Teil (5) und die Gelenkachse (31) zurückkehrt.

2. Orthese nach Anspruch 1, **dadurch gekennzeichnet, dass** sie kein Organ aufweist, das gegen die Rückseite der Hand (4) aufliegt, wie z.B. einen Gurt oder Gleichwertiges.

3. Orthese nach Anspruch 1 oder 2, **dadurch gekennzeichnet, dass** die distale Brücke (10) eine Aussparung (17) umfasst, die an ihrem distalen Rand (18) angebracht ist, wobei sich die Aussparung (17) in dem Bereich des Ellengriffels befindet, wenn die Orthese (1) getragen wird.

4. Orthese nach einem der Ansprüche 1 bis 3, **dadurch gekennzeichnet, dass** sie außerdem ein Kissen (19) umfasst, das unter dem Rückenteil - oder dem hinteren Teil - des Körpers (2) der Orthese (1) befestigt ist, unter mindestens einem Teil der distalen Brücke (10) und des Verbindungsbands (16), so dass das Kissen (19) zwischen dem Handgelenk des Benutzers und dem Körper (2) der Orthese (1) angeordnet werden kann.

5. Orthese nach Anspruch 4, **dadurch gekennzeichnet, dass** das Kissen (19) mindestens einen geprägten Vorsprung (20) umfasst, der sich im Wesentlichen quer erstreckt und hin zur Hand gedreht ist, wenn die Orthese (1) getragen wird.

6. Orthese nach einem der Ansprüche 1 bis 5, **dadurch gekennzeichnet, dass** sie außerdem Mittel zur Versteifung (22, 35, 38, 41) umfasst, die entworfen sind, um die Verformung des Körpers (2) der Orthese (1) zu verhindern, wenn ein Druck auf die palmare Auflage (7) gegenüber dem Verbindungsband (16) ausgeübt wird.

7. Orthese nach Anspruch 6, **dadurch gekennzeichnet, dass** dieMittel zur Versteifung ein starres Stück (35, 38, 41) umfasst, das auf abnehmbare Weise auf dem Verbindungsband (16) befestigt werden kann, um die elastische Verformung dieses Verbindungsbandes (16) zu verhindern.

8. Orthese nach Anspruch 6 oder 7, **dadurch gekennzeichnet, dass** die Mittel zur Versteifung mindestens ein starres Organ (22) umfassen, das auf dem proximalen Teil (5) bzw. auf dem distalen Teil (3) des Körpers (2) der Orthese (1) auf drehbare Weise um eine Achse (24), die im Wesentlichen quer liegt, montiert ist, zwischen einer aktiven Position, in der das Organ (22) an den distalen Teil (3) bzw. an den proximalen Teil (5) durch Rückhaltemittel befestigt ist, um die Verformung des Körpers (2) der Orthese (1) zu verhindern, und einer inaktiven Position, in der das Organ (22) die Verformung des Körpers (2) der Orthese (1) ermöglicht.

9. Orthese nach Anspruch 8, **dadurch gekennzeichnet, dass** das Organ eine seitliche Lasche (22) aufweist, die ein erstes Ende (23) umfasst, das auf einem seitlichen Flügel (11) des proximalen Teils (5) bzw. einem seitlichen Zweig (9) des distalen Teils (3) des Körpers (2) der Orthese (1) montiert ist, und ein zweites Ende (25), das mit Mitteln zum abnehmbaren Rückhalt auf den seitlichen Zweig (9) und auf dem seitlichen Flügel (11) in der aktiven oder inaktiven Position ausgestattet ist.

10. Orthese nach einem der Ansprüche 1 bis 9, **dadurch gekennzeichnet, dass** der Körper (2) transparent oder lichtdurchlässig ist.

11. Orthese nach einem der Ansprüche 1 bis 10, **dadurch gekennzeichnet, dass** sie außerdem einen Ellbogenschützer (28) umfasst, der die Form einer Textilhülle aufweist, wobei der Körper (2) der Orthese (1) an der Außenseite des Ellbogenschützers angeordnet ist, wenn die Orthese (1) getragen wird.

## Claims

1. An orthosis for treating muscle disorders of the elbow such as epicondylitis, capable of assisting the extensor muscles of the wrist, the orthosis (1) comprising a body (2) which is made in a single piece in semirigid material and which comprises:
- a distal portion (3) intended to be placed at the level of the hand (4), comprising a substantially transverse palm support (7) for the palm (8) of the hand, extended in the proximal direction by two substantially longitudinal lateral branches (9);
- a proximal portion (5) intended to be placed at the level of the forearm (6) comprising two substantially longitudinal lateral wings (11) intended to bear on either side of the forearm;
a strap (15) being associated with each of the lateral wings (11) of the proximal portion (5) to hold the lateral wings (11) tight against each other, the body (2) of the orthosis (1) having geometry such that the relative position of the distal and proximal portions (3,5), in the non-stressed state, corresponds to holding in extension of the wrist when the orthosis (1) is worn;
**characterized in that**
- the two lateral branches (9) of the distal portion (3) are joined at their proximal end by a substantially transverse distal bridge (10) intended to be placed over the posterior side of the wrist, and the two lateral wings (11) of the proximal portion (5) are joined at their distal end by a substantially transverse proximal bridge (12) intended to be placed over the posterior side of the forearm, the strap (15) passing underneath the anterior side (29) of the forearm;
- the body (2) comprises a back joining strap (16) extending substantially longitudinally, which connects the distal and proximal bridges (10,12) and which is elastically deformable so that:
- when pressure is applied to the palm support (7) opposite the joining strap (16), the joining strap (16) becomes deformed so as to allow pivoting of the distal portion (3) relative to the proximal portion (5) about an articulation axis (31) which is substantially transverse and parallel to the flexion-extension axis (30) of the wrist when the orthosis (1) is worn;
- and when said pressure is released, the body (2) returns to the non-stressed state via elasticity by pivoting of the distal portion (3) relative to the proximal portion (5) about said articulation axis (31).

2. The orthosis according to claim 1, **characterized in that** it is free of any member bearing on the dorsal side of the hand (4) such as a strap or equivalent.

3. The orthosis according to claim 1 or 2, **characterized in that** the distal bridge (10) comprises a notch (17) arranged on its distal edge (18), the said notch (17) being located in the region of the ulnar styloid when the orthosis (1) is worn.

4. The orthosis according to one of claims 1 to 3, **characterized in that** it further comprises a cushion (19) attached underneath the dorsal - or posterior - part of the body (2) of the orthosis (1), underneath at least part of the distal bridge (10) and of the joining strap (16), so that the cushion (19) is able to be inserted between the user's wrist and the body (2) of the orthosis (1).

5. The orthosis according to claim 4, **characterized in that** the cushion (19) comprises at least one raised portion (20) extending substantially transversely and facing the hand when the orthosis (1) is worn.

6. The orthosis according to one of claims 1 to 5, **characterized in that** it further comprises rigidifying means (22, 35, 38, 41) designed so that they can prevent deformation of the body (2) of the orthosis (1) when pressure is applied to the palm support (7) opposite the joining strap (16).

7. The orthosis according to claim 6,**characterized in that** the rigidifying means comprise a rigid part (35, 38, 41) able to be removably fastened onto the joining strap (16) so as to prevent elastic deformation of this joining strap (16).

8. The orthosis according to claim 6 or 7, **characterized in that** the rigidifying means comprise at least one rigid member (22) mounted on the proximal portion (5), respectively on the distal portion (3) of the body (2) of the orthosis (1) so as to pivot about a substantially transverse axis (24) between an active position in which the member (22) is fastened onto the distal portion (3), respectively onto the the proximal portion (5), by retaining means, to prevent deformation of the body (2) of the orthosis (1), and an inactive position in which the member (22) allows deformation of the body (2) of the orthosis (1).

9. The orthosis according to claim 8, **characterized in that** the member comprises a lateral tab (22) having a first end (23) which is mounted on a lateral wing (11) of the proximal portion (5), respectively a lateral branch (9) of the distal portion (3), of the body (2) of the orthosis (1), and a second end (25) which is provided with removable retaining means on said lateral branch (9) and said lateral wing (11), in active or inactive position.

10. The orthosis according to one of claims 1 to 9, **characterized in that** the body (2) is transparent or translucent.

11. The orthosis according to one of claims 1 to 10,**characterized in that** it further comprises an elbow support (28) in the form of a fabric sleeve, the body (2) of the orthosis (1) being placed over the elbow support when the orthosis (1) is worn.
